# EUROPEAN PATENT SPECIFICATION

(11) **EP 1 625 865 B1**
(45) Date of publication and mention of the grant of the patent: **04.03.2009**
(21) Application number: 04254805.7
(22) Date of filing: 10.08.2004
(51) Int. Cl.: A61M 5/32

(54) **Disposable syringe with a retractable needle**
Einweg Spritze mit einziehbarer Nadel
Seringue jetable à aiguille rétractable

(43) Date of publication of application: 15.02.2006
(73) Proprietor: Shue, Ming-Jeng, Hsi Dist, Taichung City (TW); Huang, Deborah, Taichung City (TW); Shue, Phillip, Hsi District, Taichung City (TW)
(72) Inventor: Shue, Ming-Jeng, Hsi Dist, Taichung City (TW); Huang, Deborah, Taichung City (TW); Shue, Phillip, Hsi District, Taichung City (TW)
(74) Representative: Mounteney, Simon James

(56) References cited:
- EP-A- 1 421 962
- US-A- 5 084 018
- US-A- 5 211 628
- US-A1- 2003 236 501

## Description

This invention relates to a disposable syringe, more particularly to a disposable syringe with a friction diminishing means for facilitating retraction of a needle into a tubular plunger.

Conventional syringes, especially those with a sharp needle, have to be disposed safely after injection. Therefore, there are many syringes with a retractable needle that is retracted into a plunger when the plunger reaches the end of its stroke. However, it is desirable to improve the steady retracted movement of the syringe and the variety of the diameter of the syringe barrel.

A disposable syringe disclosed in EP 1,421,962 A1 includes a tubular barrier member retained in a syringe barrel such that a tubular needle seat for fixing a needle cannula is retained on the barrier member. The needle seat abuts against an abutment wall in the barrel while the needle cannula is disposed outwardly of the barrel. A plunger is slidable in the barrel for injection, and has a seal member to seal a cavity confined in a plunger body and containing a fluid at a reduced pressure. After injection is completed, when a forward pushing force applied to the plunger body moves the barrier member, the needle seat is released, and the seal member is ruptured, so that the needle seat and the used needle cannula are suctioned into the cavity due to the pressure difference between the ambient atmosphere and the reduced pressure.

Another syringe disclosed in U.S. Publication No. 2003/0236501 A1 includes a barrel, a hollow fluid dispensing needle which projects from the barrel, means for retracting the needle after use from the projecting position to a retracted, inaccessible position, and a sheath which is removably engageable with the syringe to sheath the needle and provides an interior zone for communication with the internal bore of the needle. The sheath is apertured to admit liquid to the zone whereby liquid drawn through the needle into the syringe first passes into the zone.

The object of the present invention is to provide a disposable syringe which permits steady retraction of a plunger and which ensures successful retraction of a needle assembly.

According to this invention, the disposable syringe includes a needle cannula, a tubular needle seat, a barrel, a tubular grip member, a tubular plunger, a coupling member and a biasing member.

The tubular needle seat includes a front hub portion which is disposed to fix the needle cannula therein, a gripped portion which extends from the front hub portion in a longitudinal direction, and a rear anchoring portion which is disposed opposite to the front hub portion in the longitudinal direction.

The barrel has an inner surrounding barrel surface which surrounds an axis in the longitudinal direction and which defines a passage therein. The passage has rearward and forward openings opposite to each other in the longitudinal direction. The inner surrounding barrel surface includes a larger-diameter portion and a smaller-diameter portion which are disposed proximate to the rearward and forward openings, respectively. The larger-diameter portion has a retaining area which is spaced apart from the smaller-diameter portion in the longitudinal direction. The smaller-diameter portion includes a retaining region which is disposed proximate to the larger-diameter portion and which is configured to retain the front hub portion thereat when the needle seat is in a position of use, and a friction diminishing region which extends from the retaining region toward the forward opening and which terminates at a shoulder abutment. The shoulder abutment defines a communicating hole which permits passage of the needle cannula therethrough, and is spaced apart from the front hub portion along the axis in the position of use.

The tubular grip member, in the position of use, is disposed to bring the gripped portion into engagement with the retaining area by virtue of a first frictional force generated therebetween.

The tubular plunger is disposed to be movable in the passage along the larger-diameter portion. The plunger has a front opened end wall which is movable to abut against the grip member, a rear opened end wall which is disposed opposite to the front opened end wall and which extends outwardly of the rearward opening so as to be manually operable, and an intermediate surrounding wall which is interposed between the front and rear opened end walls and which defines an accommodation chamber.

The coupling member has a surrounding retained portion which surrounds the axis and which is disposed in the accommodation chamber to be in frictional engagement with the intermediate surrounding wall by virtue of a second frictional force, and an anchored portion which is disposed adjacent to the front opened end wall in the position of use, which confronts the rear anchoring portion, and which is engageable with the rear anchoring portion by a holding force when the coupling member is moved forwardly towards the forward opening. Thus, when the grip member is pushed by virtue of forward movement of the plunger against the first frictional force to move the front hub portion past the friction diminishing region to abut against the shoulder abutment, a pushing force subsequently applied to the plunger causes the anchored portion to rub against the rear anchoring portion, which remains unmoved in place due to engagement of the front hub portion with the shoulder abutment, so that the anchored portion is engaged with the rear anchoring portion and is moved relative to the intermediate surrounding wall towards the rear opened end wall so as to release the surrounding retained portion from the intermediate surrounding wall, thereby enabling the anchored portion to be moved from the position of use to a retracted position where the anchored portion is disposed closer to the rear opened end wall, and where the needle seat and the needle cannula are received in the accommodation chamber.

The biasing member is disposed to bias the anchored portion towards the retracted position.

Other features and advantages of the present invention will become apparent in the following detailed description of the preferred embodiments of the invention, with reference to the accompanying drawings, in which:
Fig. 1 is an exploded sectional view of the first preferred embodiment of a disposable syringe according to this invention;
Fig. 2 is a sectional view of the first preferred embodiment in a state of use;
Figs. 3 to 5 are fragmentary sectional views of the first preferred embodiment, showing a coupling member in three different states;
Fig. 6 is a sectional view of the first preferred embodiment in a retracted state;
Fig. 7 is a sectional view of the second preferred embodiment of a disposable syringe according to this invention;
Figs. 8 and 9 are sectional views of the third preferred embodiment of a disposable syringe according to this invention in a state of use and in a retracted state, respectively;
Fig. 10 is a sectional view of the fourth preferred embodiment of a disposable syringe according to this invention;
Figs. 11 is a sectional view of the fifth preferred embodiment of a disposable syringe according to this invention in a state of use;
Fig. 12 is a sectional view of the fifth preferred embodiment, showing the state in which a needle seat passes by a friction diminishing region;
Fig. 13 is a sectional view of the fifth preferred embodiment in a retracted state;
Figs. 14 and 15 are sectional views of the sixth and seventh preferred embodiments of a disposable syringe according to this invention, respectively;
Figs. 16 and 17 are sectional views of the eighth preferred embodiment of a disposable syringe according to this invention in a state of use and in a retracted state, respectively;
Fig. 18 is a sectional view of the ninth preferred embodiment of a disposable syringe according to this invention in a state of use;
Fig. 19 is a fragmentary sectional view of the ninth preferred embodiment, showing the state in which a needle seat passes by a friction diminishing region;
Fig. 20 is a fragmentary sectional view of the tenth preferred embodiment of a disposable syringe according to this invention in a state of use;
Fig. 21 is a sectional view of the tenth preferred embodiment in a retracted state; and
Figs. 22 to 25 are sectional views of the eleventh, twelfth, thirteenth and fourteenth preferred embodiments of a disposable syringe according to this invention, respectively.

Before the present invention is described in greater detail, it should be noted that same reference numerals have been used to denote like elements throughout the specification.

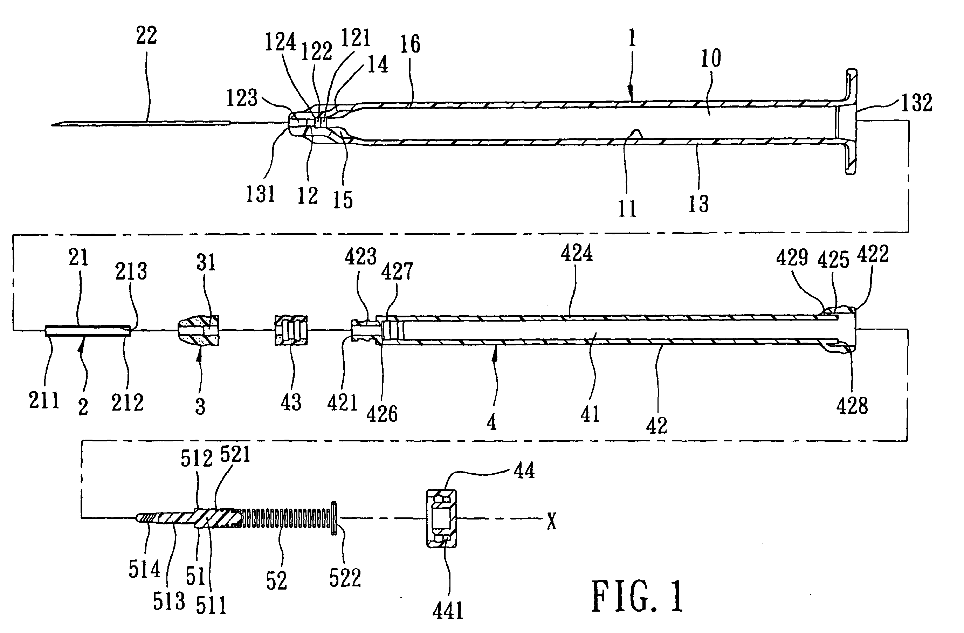

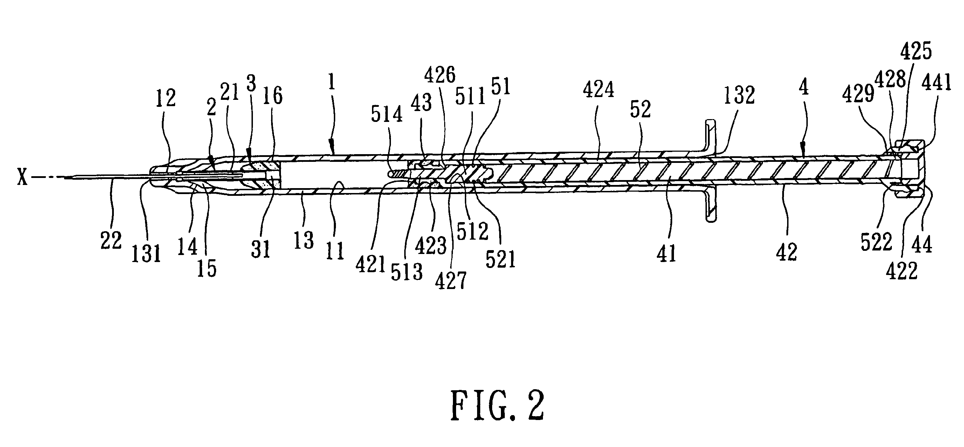

Referring to Figs. 1 and 2, the first preferred embodiment of a disposable syringe according to the present invention is shown to comprise a needle assembly 2, a barrel 1, a tubular grip member 3, a tubular plunger 4, a coupling member 51, and a biasing member 52.

The needle assembly 2 includes a needle cannula 22 and a tubular needle seat 21. The needle seat 21 is in the form of a hard thin wall tube, such as a metal or carbon fiber tube which includes a front segment 211 to serve as a front hub portion 211 that is disposed to fix the needle cannula 22 therein, and a rear segment that extends from the front segment along an axis (X) in a longitudinal direction. The rear segment has outer and inner segment surfaces opposite to each other in radial directions relative to the axis (X) to serve as a gripped portion 212 and a rear anchoring portion 213, respectively.

The barrel 1 has an inner surrounding barrel surface 13 which surrounds the axis (X) in the longitudinal direction and which defines a passage 10 therein. The passage 10 has rearward and forward openings 132,131 which are disposed opposite to each other in the longitudinal direction. The inner surrounding barrel surface 13 includes a larger-diameter portion 11 and a smaller-diameter portion 12 which are disposed proximate to the rearward and forward openings 132, 131, respectively, anannular shoulder 14 which converges gradually from the larger-diameter portion 11 to the smaller-diameter portion 12, and a plurality of ribs 15 which are formed on an inner surface of the shoulder 14 to retain a part of the front hub portion 211 of the needle seat 21. The larger-diameter portion 11 has a retaining area 16 which is in the form of an annular protrusion and which is spaced apart from the smaller-diameter portion 12 in the longitudinal direction.

The smaller-diameter portion 12 includes a retaining region 121 which is disposed in connection with the ribs 15 and which is configured to retain a part of the front hub portion 211 of the needle seat 21 thereat when the needle seat 21 is in a position of use, and a friction diminishing region 122 which extends from the retaining region 121 toward the forward opening 131 and which terminates at a shoulder abutment 124. The passage 10 at the friction diminishing region 122 has a diameter larger than that of the passage 10 at the retaining region 121. The shoulder abutment 124 defines a communicating hole 123 which is configured to permit passage of the needle cannula 22 therethrough, and is spaced apart from the front hub portion 211 along the axis (X) in the position of use.

In the position of use, the tubular grip member 3 is disposed, to bring the gripped portion 212 of the needle seat 21 into engagement with the retaining area 16 by virtue of a first frictional force generated therebetween. In particular, the grip member 3 is in fluid-tight and frictional engagement with the retaining area 16, and has an axially extending hole 31 for receiving the gripped portion 212.

The tubular plunger 4 is disposed to be movable in the passage 10 along the larger-diameter portion 11. The plunger 4 has a front opened end wall 421 which is movable to abut against the grip member 3, a rear opened end wall 422 which is disposed opposite to the front opened end wall 421 and which extends outwardly of the rearward opening 132 so as to be manually operable, and an intermediate surrounding wall 42 which is interposed between the front and rear opened end walls 421,422 and which defines an accommodation chamber 41. The intermediate surrounding wall 42 has a smaller front segment 423 and a larger rear segment 424 disposed proximate to the front and rear opened end walls 421, 422 , respectively, to form a shoulder 426 therebetween. An enlarged terminal segment 425 is disposed between the larger rear segment 424 and the rear opened end wall 422, and is formed with a vent hole 429 so as to be in fluid communication with the ambient atmosphere. An annular rib 428 is disposed between the larger rear segment 424 and the enlarged terminal segment 425, and extends in the longitudinal direction. An end cap 44 is disposed to cover the rear opened end wall 422, and has an annular groove 441 for engagement with the enlarged terminal segment 425. In addition, an annular groove 427 is formed in an inner peripheral surface of the larger rear segment 424 proximate to the shoulder 426. A seal ring 43, which is made of a deformable material, is sleeved retainingly over an outer peripheral surface of the smaller front segment 423 so as to be slidable on and to be in fluid-tight frictional engagement with both the larger-diameter portion 11 of the inner surrounding barrel surface 13 and the coupling member 51.

The coupling member 51 has a surrounding retained portion 512 which surrounds the axis (X) and which is in the form of an annular protrusion disposed to be in frictional engagement with the annular groove 427 in the intermediate surrounding wall 42 by virtue of a second frictional force, an anchored portion 514 which extends forwardly of the front opened end wall 421 in the position of use to confront the rear anchoring portion 213 so as to be engageable with the rear anchoring portion 213 by a holding force when the coupling member 51 is moved forwardly towards the forward opening 131, a retained shank portion 513 which is disposed between the surrounding retainedportion 512 and the anchored portion 514 and which extends in the accommodation chamber 41 at the smaller front segment 423 so as to stabilize the coupling member 51 at the smaller front segment 423, and a shank portion 511 which extends from the surrounding retained portion 512 distal from the anchored portion 514.

The biasing member 52 is a coiled spring, and has a front spring end 521 which engages the shank portion 511, and a rear spring end 522 which is retained between the annular rib 428 and the intermediate surrounding wall 42 such that the coiled spring 52 is tensioned when the surrounding retained portion 512 is in frictional engagement with the annular groove 427 by virtue of the second frictional force, as shown in Fig. 2.

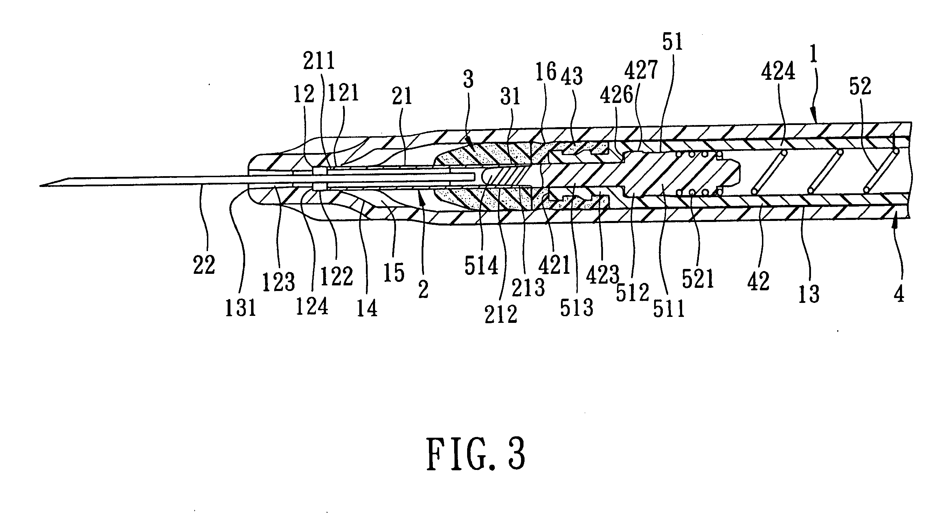

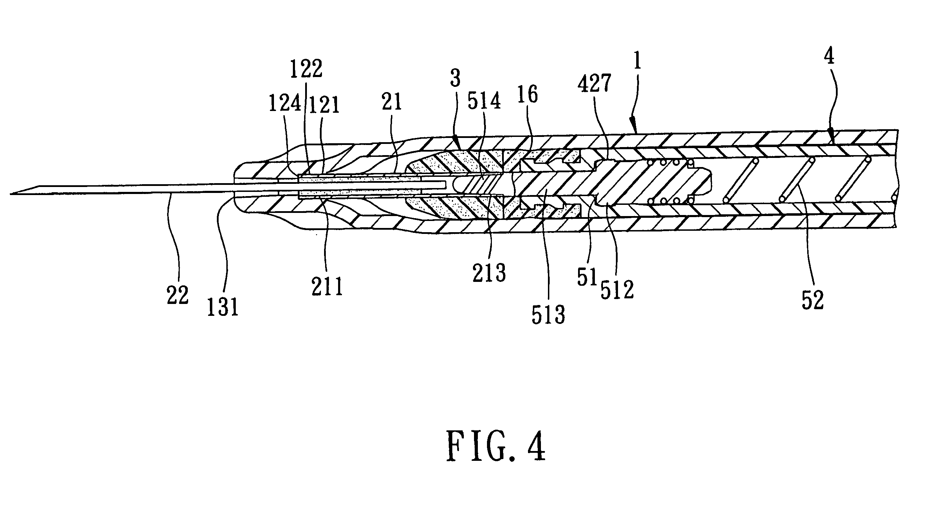

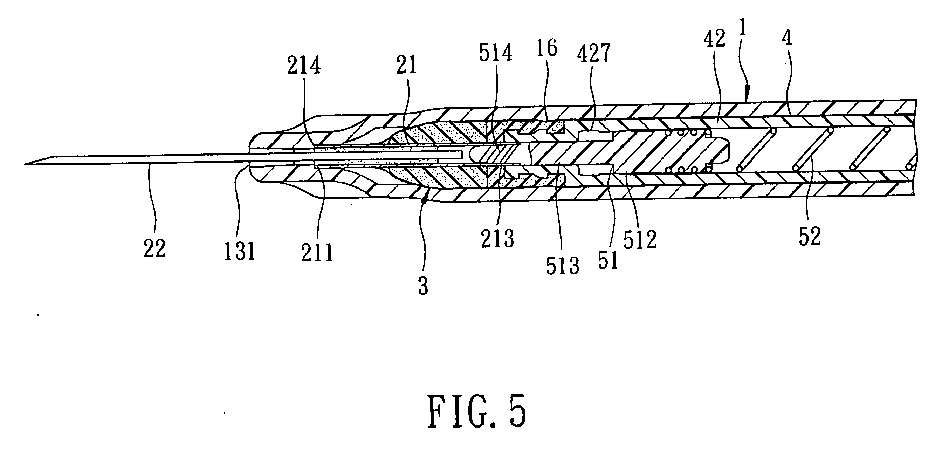

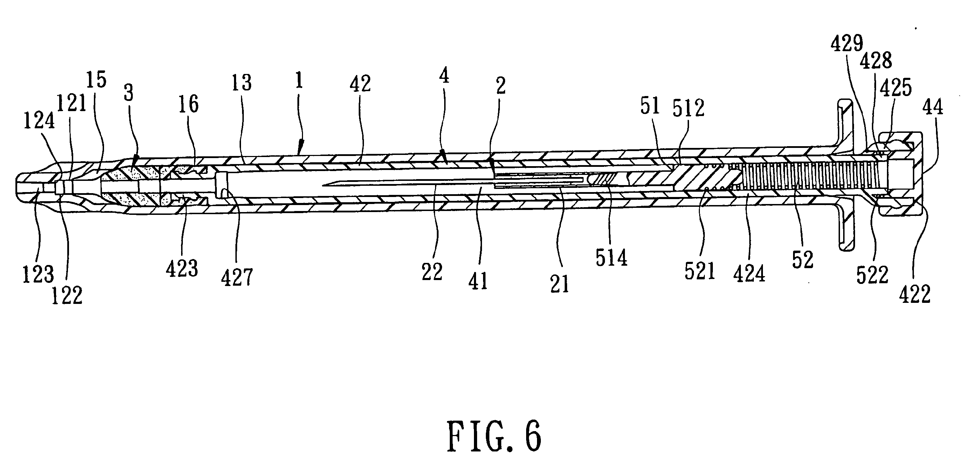

After completion of an injection course, the plunger 4 is pressed forwardly by a pushing force to permit abutment of the seal ring 43 against the grip member 3. At this time, the anchored portion 514 is extended into the axially extending hole 31. Subsequently, with reference to Figs. 3 and 4, a manual pushing force is further applied to the plunger 4 to push the grip member 3 forward against the first frictional force (i.e., the frictional engagement between the grip member 3 and the retaining area 16) such that the anchored portion 514 is partially extended into and engaged with the rear anchoring portion 213, and such that the front hub portion 211 moves past the friction diminishing region 122 to abut against the shoulder abutment 124. Meanwhile, the movement of the front hub portion 211 past the friction diminishing region 122 results in reduced friction therebetween. Therefore, as shown in Fig. 5, when a pushing force is subsequently applied to the plunger 4 to cause the anchored portion 514 to rub against the rear anchoring portion 213, which remains unmoved due to engagement of the front hub portion 211 with the shoulder abutment 124, the anchored portion 514 is engaged with the rear anchoring portion 213 and is moved relative to the intermediate surrounding wall 42 towards the rear opened end wall 422 so as to release the surrounding retained portion 512 from the annular groove 427 in the intermediate surrounding wall 42, thereby enabling the anchored portion 514 to be moved by the biasing force of the coiled spring 52 from the position of use to a retracted position where the anchored portion 514 is disposed closer to the rear opened end wall 422 and where the needle assembly 2 (i.e. the needle seat 21 and the needle cannula 22) is received in the accommodation chamber 41, as shown in Fig. 6.

It is noted that with the provision of the friction diminishing region 122, which provides a space of triggering action for retraction of the needle assembly 2, the front hub portion 211 can be pushed forwards relative to the smaller-diameter portion 12 so that the friction between the front hub portion 211 and the smaller-diameter portion 12 to be overcome in the course of retraction of the needle assembly 2 into the accommodation chamber 41 by the coiled spring 52 can be reduced. In other words, the substantially continuing forward movement of the needle seat 21 relative to the smaller-diameter portion 12 after the injection course and its subsequently rearward movement due to retraction of the needle assembly 2 by the coiled spring 52, transform the static friction between the front hub portion 211 and the smaller-diameter portion 12 into a kinetic friction, which can be easily overcome by the predetermined biasing force of the coiled spring 52 in the course of retraction of the needle assembly 2 into the accommodation chamber 41, thereby ensuring successful and smooth retraction of the needle assembly 2.

It is further noted that since the needle seat 21 is in the form of a metal tube, it can be configured to have a relatively small diameter. Thus, the needle cannula 22 can be formed to have a relatively small diameter, as shown in Figs. 1 to 5, so as to be adapted for injecting medication of a very small volume, such as 1 ml. Likewise, the barrel 1 can be configured to have a smaller diameter with a relatively compensatory elongation of the length of the barrel for a unit medication volume. Thus, the spacing of graduations (not shown) marked on the barrel 1 of extremely small volume, such as 1 cc., and smaller ones, can be relatively large to facilitate accurate reading of the volume of medication in injection course.

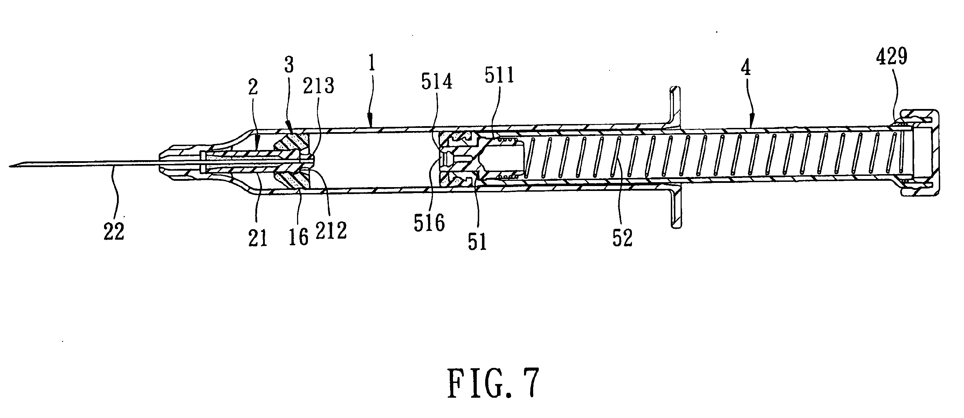

Referring to Fig. 7, the second preferred embodiment of a disposable syringe according to this invention is similar to the first preferred embodiment in construction, but is adapted for injecting medication of a general volume, such as 3ml, 5ml, or more. That is, the barrel 1, the tubular plunger 4 and the tubular needle seat 21 have relatively large diameters. The tubular needle seat 21 is in the form of a plastic injecting tube, and has a rear anchoring portion 213 which extends rearwardly from the gripped portion 212 along the axis (X). In addition, the coupling member 51 has an anchored portion 514 which has an engaging recess 516 that is configured to grip the rear anchoring portion 213 with a holding force when the needle seat 21 is placed in the retracted position.

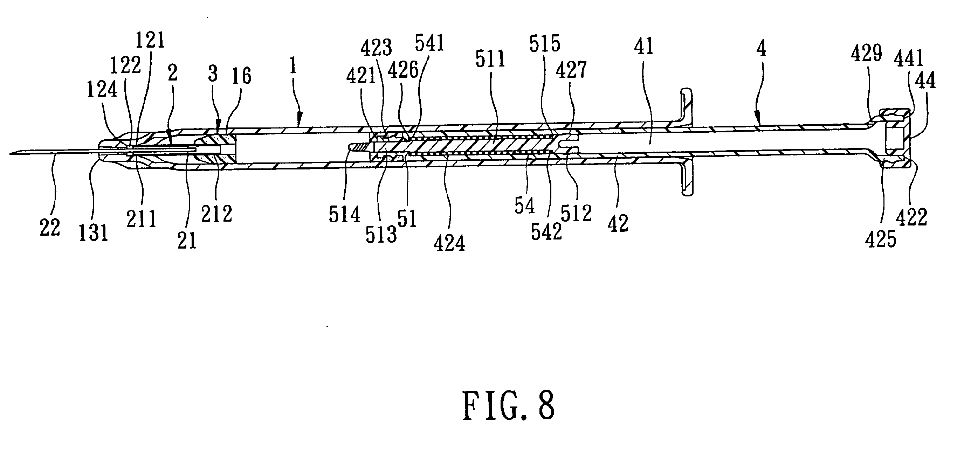

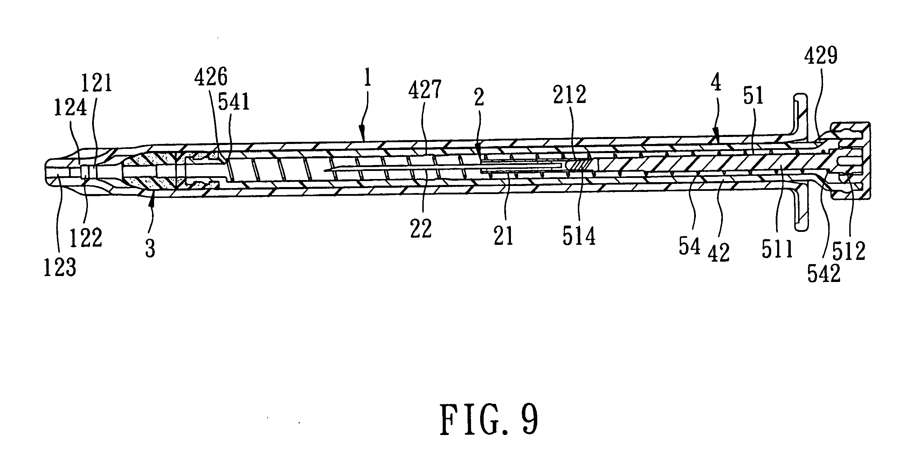

Referring to Figs. 8 and 9, the third preferred embodiment of a disposable syringe according to this invention is similar to the first preferred embodiment in construction, except that the coupling member 51 has a shank portion 511 which is interposed between the anchored portion 514 and the surrounding retained portion 512. In addition, the intermediate surrounding wall 42 of the tubular plunger 4 and the shank portion 511 respectively have an annular shoulder 426 and a flange 515 which are respectively proximate to the front opened end wall 421 of the plunger 4 and distal from the anchored portion 514 and which are spaced apart from each other in the longitudinal direction so as to define a biasing member receiving space therebetween. The biasing member 54 is a coiled spring which has front and rear spring ends 541,542 abutting against the annular shoulder 426 and the flange 515, respectively, such that the coiled spring is compressed when the surrounding retained portion 512 is in frictional engagement with the annular groove 427 in the intermediate surrounding wall 42 by virtue of the second frictional force.

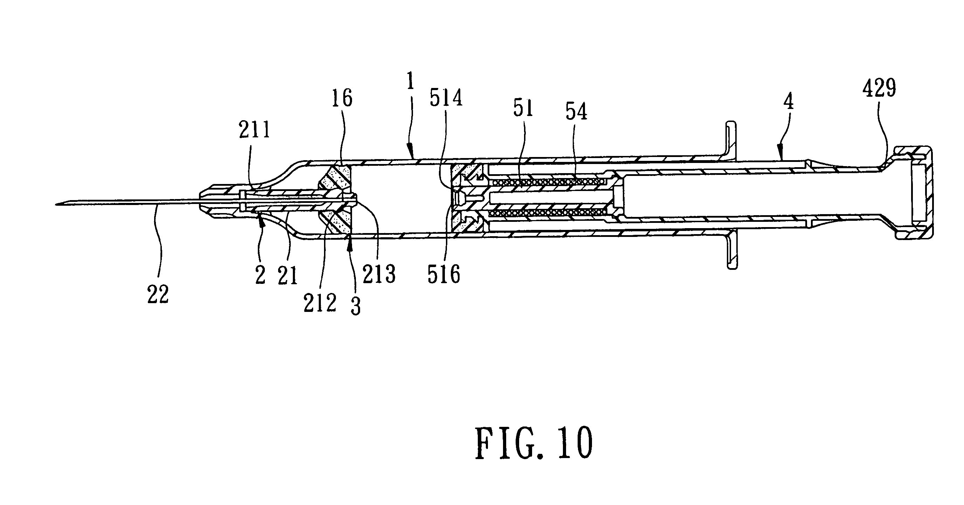

Referring to Fig. 10, the fourth preferred embodiment of a disposable syringe according to this invention is similar to the third preferred embodiment in construction, but is adapted for injecting medication of a general volume, such as 3 ml, 5 ml, or more. That is, the barrel 1, the tubular plunger 4 and the tubular needle seat 21 have relatively larger diameters. The tubular needle seat 21 is in the form of a plastic injecting tube, and has a rear anchoring portion 213 which extends rearwardly from the gripped portion 212 along the axis (X). In addition, the coupling member 51 has an anchored portion 514 which has an engaging recess 516 which is configured to grip the rear anchoring portion 213 with a holding force when the needle seat 21 is placed in the retracted position.

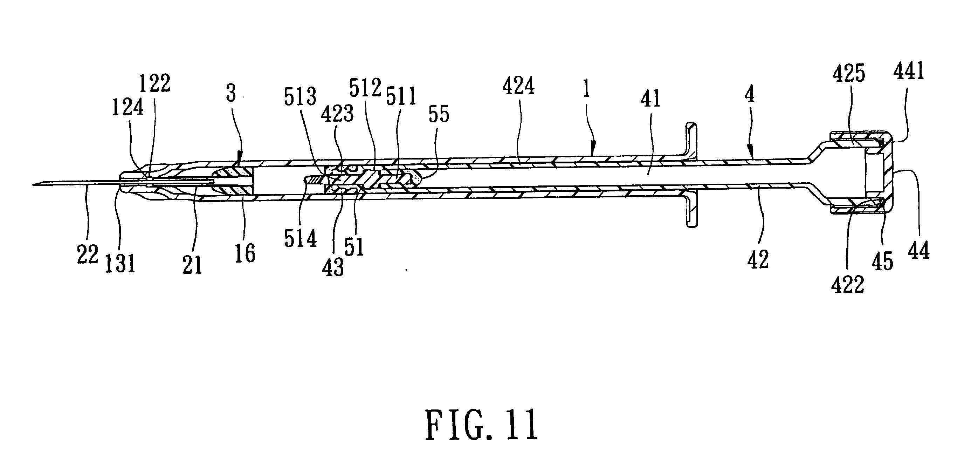

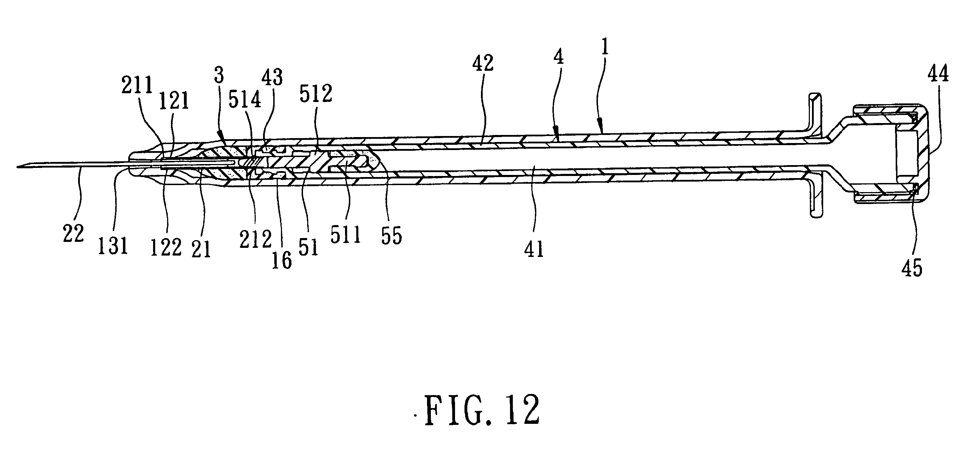

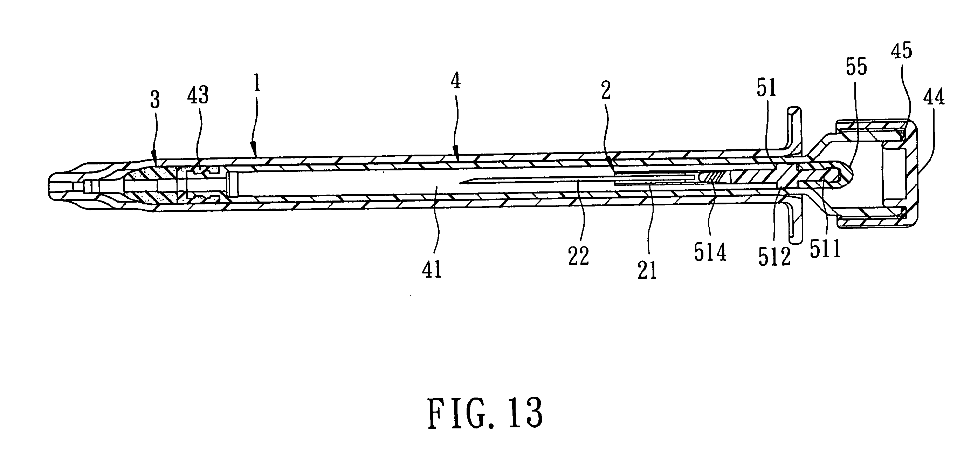

Referring to Figs. 11 to 13, the fifth preferred embodiment of a disposable syringe according to this invention is similar to the first preferred embodiment in construction. The difference resides in that a seal ring member 45, such as an O-ring, is disposed between the rear opened end wall 422 and the annular groove 441 in the end cap 44 to establish fluid-tight engagement therebetween. The enlarged terminal segment 425 is not formed with the vent hole described in the first preferred embodiment. In addition, instead of the coiled spring 52, 54 described above, the biasing member includes a fluid, such as air, which is contained in the accommodation chamber 41 in the tubular plunger 4 at a relatively reduced pressure, and a sealing member 55 which is sleeved on the shank portion 511 of the coupling member 51 to provide a seal between the coupling member 51 and the intermediate surrounding wall 42 so as to trap the fluid in the accommodation chamber 41. Thus, when the surrounding retained portion 512 is released from the intermediate surrounding wall 42, the anchored portion 514 is suctioned to the retracted position due to a pressure difference between the ambient atmosphere and the reduced pressure.

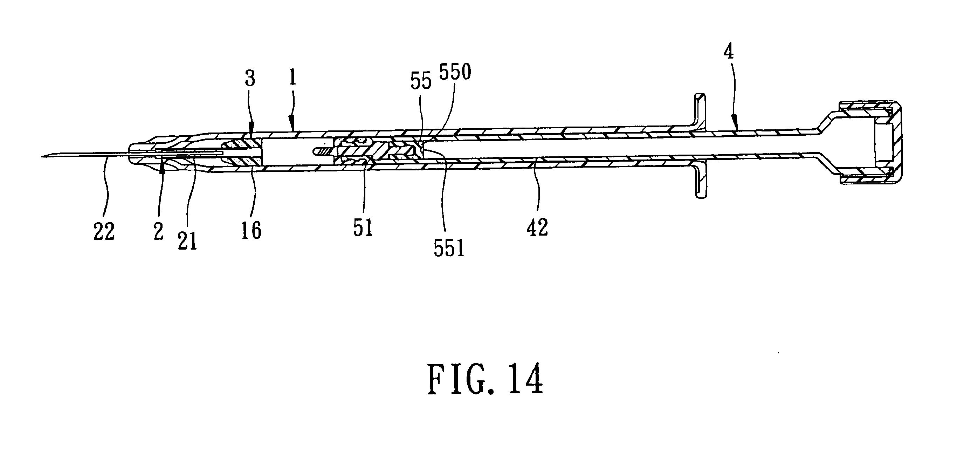

Further, referring to Fig. 14, in the sixth preferred embodiment of a disposable syringe according to this invention, which is similar to the fifth preferred embodiment, the sealing member 55 extends in the longitudinal direction to terminate at a rear end wall 550 which is disposed rearwardly of the coupling member 51 and which has a central recess 551 extending inwardly and along the axis (X) so as to enhance deformability of the rear end wall 550 in radial directions, thereby enhancing sealing attachment of the sealing member 55 with the intermediate surrounding wall 42.

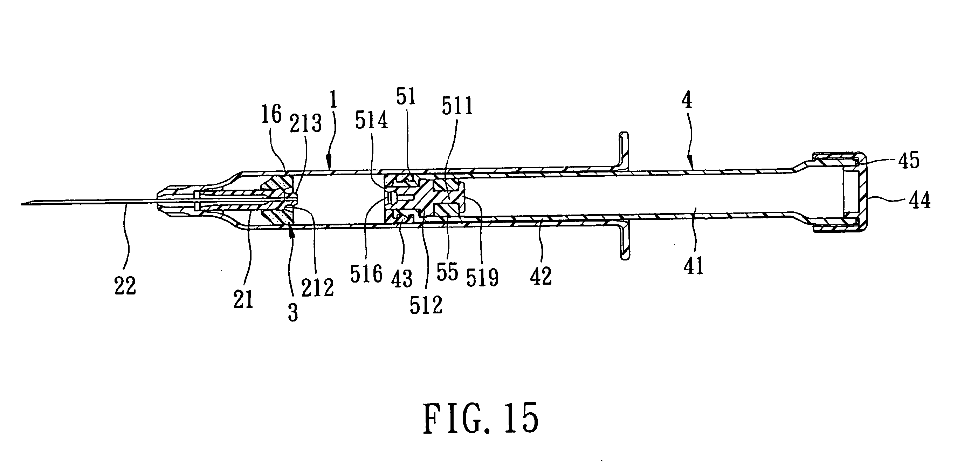

Referring to Fig. 15, the seventh preferred embodiment of a disposable syringe according to this invention is similar to the fifth preferred embodiment in construction, but is adapted for injecting medication of a general volume, such as 3 ml, 5 ml, or more. The tubular needle seat 21 is in the form of a plastic injecting tube, and has a rear anchoring portion 213 which extends rearwardly from the gripped portion 212 along the axis (X) so as to engage an engaging recess 516 in the anchored portion 514 of the coupling member 51 with a holding force when the needle seat 21 is placed in the retracted position. In addition, the coupling member 51 has a shank portion 511 extending rearwardly from the surrounding retained portion 512 to terminate at an enlarged head portion 519. The sealing member 55 is sleeved on the shank portion 511, and is interposed between the head portion 519 and the surrounding retained portion 512 so as to be deformed radially for enhancing the sealing attachment with the intermediate surrounding wall 42.

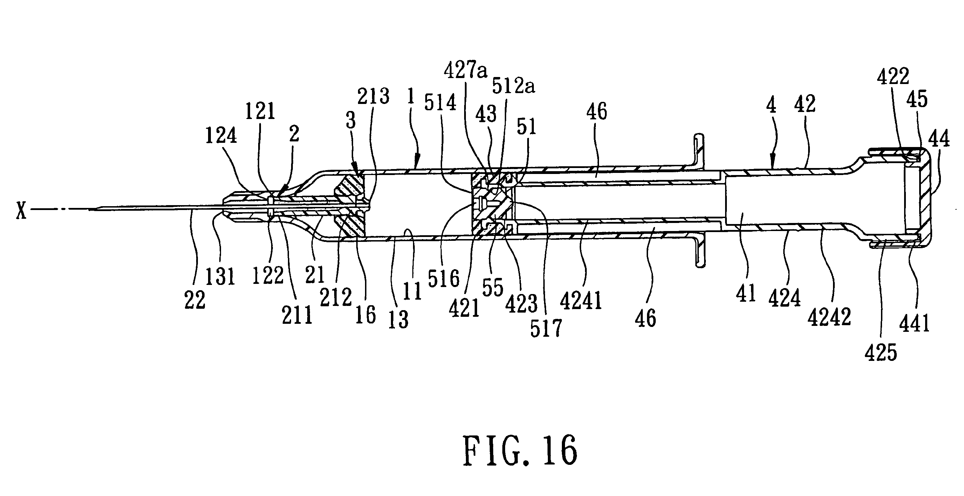

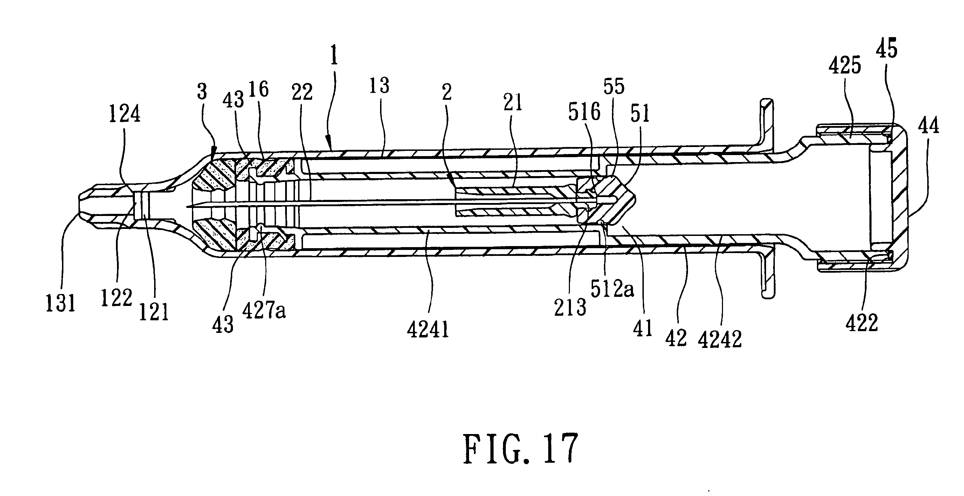

Referring to Figs. 16 and 17, the eighth preferred embodiment of a disposable syringe according to this invention is similar to the seventh preferred embodiment in construction, and is adapted for injecting medication of a general volume, such as 3 ml, 5 ml, or more. In this embodiment, the larger rear segment 424 of the intermediate surrounding wall 42 of the tubular plunger 4 includes front andrearwall segments 4241, 4242 which are disposed proximate to the front and rear opened end walls 421, 422, respectively, and which have smaller and larger outer peripheral surfaces, respectively. The plunger 4 further includes a plurality of rib fins 46 which are formed on the smaller outer peripheral surface of the front wall segment 4241 and which are flush with the larger outer peripheral surface of the rear wall segment 4242 so as to guide movement of the plunger 4 relative to the larger-diameter portion 11 of the inner surrounding barrel surface 13 while minimizing frictional force therebetween. The smaller front segment 423 and the coupling member 51 respectively have an annular protrusion 427a and an annular groove 512a which engage each other to provide the second frictional force. The coupling member 51 further has a tapered rear portion 517 opposite to the engaging recess 516 so as to establish fluid-tight engagement with the smaller front segment 423. The sealing member 55 is formed integrally with the coupling member 51 and is disposed between the tapered rear portion 517 and the annular groove 512a.

When the surrounding retainedportion (the annular groove 512a) is released from the intermediate surrounding wall 42 (the annular protrusion 427a), the coupling member 51 is received in the accommodation chamber 41 at the front wall segment 4241, and is held in fluid-tight contact with the front wall segment 4241, thereby facilitating sliding of the coupling member 51, as well as the needle assembly 2, to the retracted position.

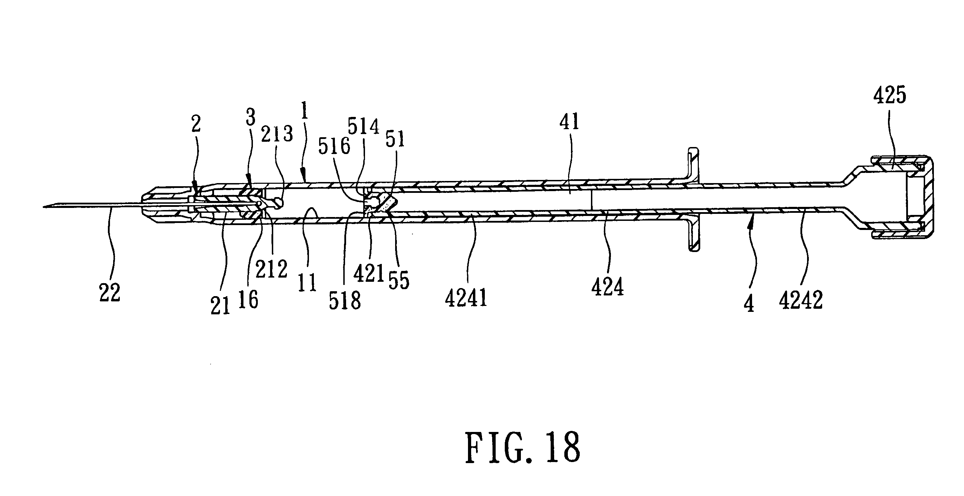

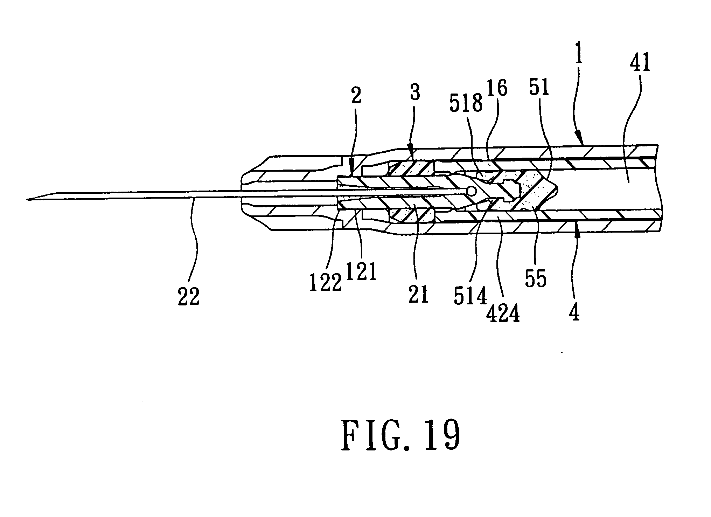

Referring to Figs. 18 and 19, the ninth preferred embodiment of a disposable syringe according to this invention is similar to the eighth preferred embodiment in construction, but is adapted for injecting medication of an extremely small injection volume, such as 1ml. The sealing member 55 is formed integrally with the coupling member 51, is made from a deformable material, and surrounds the anchored portion 514. In addition, the coupling member 51 has an annular front edge 518 which is slidable on and is in fluid-tight engagement with the larger-diameter portion 11 of the barrel 1 so as to serve as the seal ring 43 in the previous preferred embodiment. When the coupling member 51 is placed in the retracted position, the annular front edge 518 is deformed and converged to be retracted into the accommodation chamber 41.

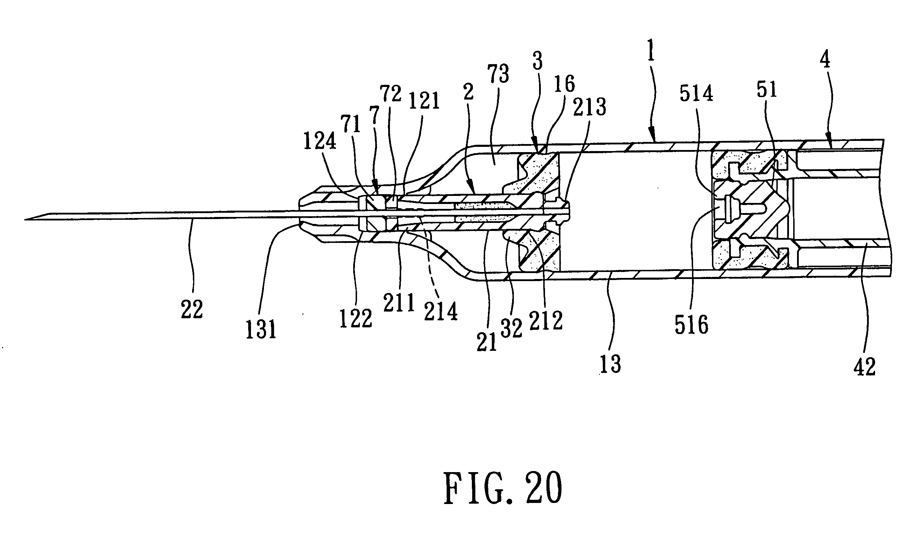

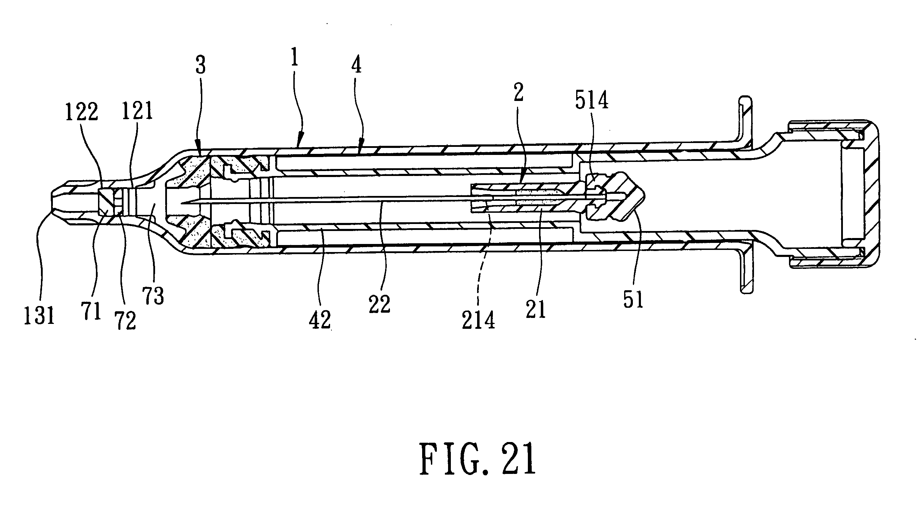

Referring to Figs. 20 and 21, the tenth preferred embodiment of a disposable syringe according to this invention is similar to the eighth preferred embodiment in construction. In this embodiment, a sealing unit 7 is further disposed to be in fluid-tight engagement with the friction diminishing region 122, and is spaced apart from the shoulder abutment 124 to define a space of triggering action for retraction of the needle assembly 2. The sealing unit 7 includes an elastomeric plug 71 and a ring plate 72 abutting against each other. The tubular grip member 3 is formed with a deformable sealing portion 32 which is in fluid-tight engagement with the gripped portion 212 of the needle seat 21 so as to form a fluid-tight compressible chamber 73. The compressible chamber 73 is filled with a fluid. The front hub portion 211 of the needle seat 21 is disposed to abut against the sealing unit 7, and has a plurality of through holes 214 formed therethrough so as to be in fluid communication with the compressible chamber 73. Thus, when the tubular grip member 3 is pushed forwardly by virtue of forward movement of the plunger 4 to move the front hub portion 211 past the friction diminishing region 122, the fluid in the compressible chamber 73 is squeezed through the through holes 214, thereby generating a pressure force in the longitudinal direction that depresses the surrounding gripped portion 212 rearwardly to help thrust the anchored portion 514 rearward to the retracted position.

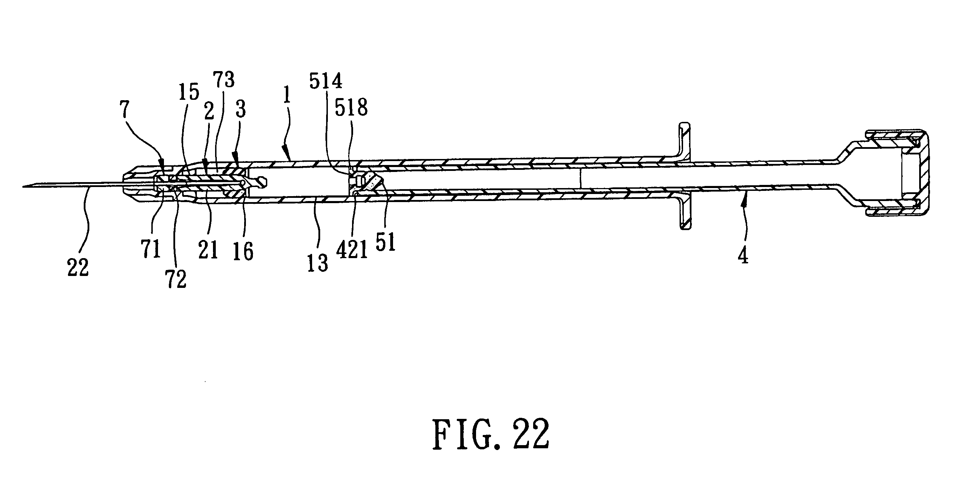

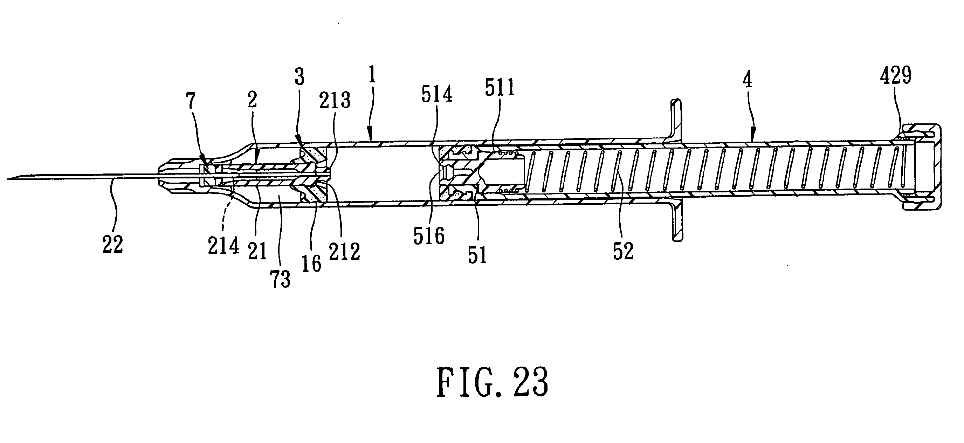

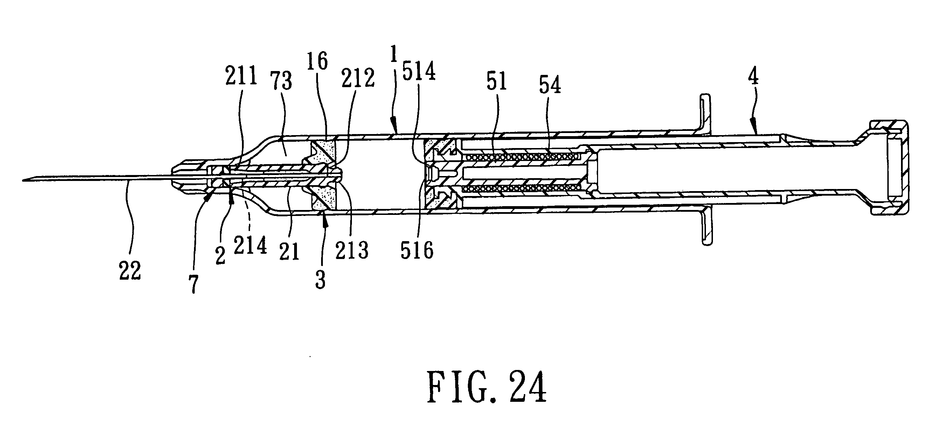

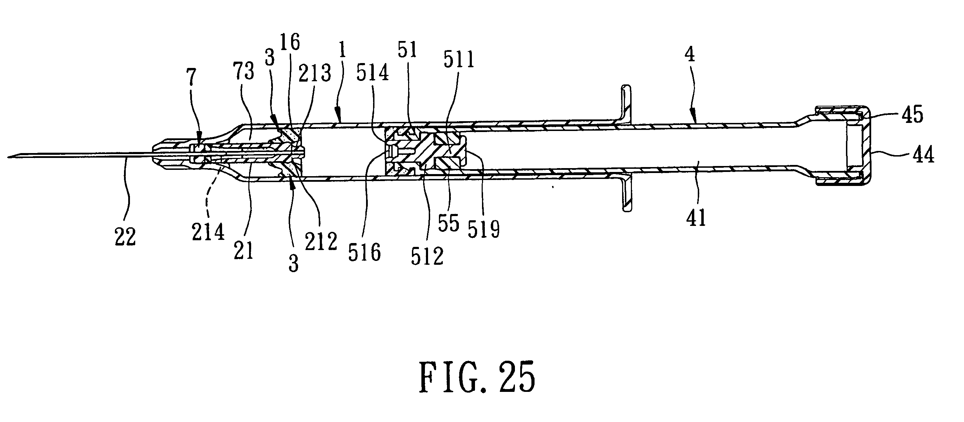

Referring to Figs. 22 to 25, the eleventh, twelfth, thirteenth and fourteenth preferred embodiments of a disposable syringe according to this invention are respectively similar to the ninth, second, fourth and sixth preferred embodiments in construction. Besides, in each of these embodiments, the disposable syringe further comprises a sealing unit 7 which is the same as that of the tenth preferred embodiment, and which is disposed to be in fluid-tight engagement with the friction diminishing region 122.

## Claims

1. A disposable syringe comprising:
a needle cannula (22);
a tubular needle seat (21) including a front hub portion (211) which is disposed to fix said needle cannula (22) therein, a gripped portion (212) which extends from said front hub portion (211) in a longitudinal direction, and a rear anchoring portion (213) which is disposed opposite to said front hub portion (211) in the longitudinal direction;
a barrel (1) having an inner surrounding barrel surf ace (13) which surrounds an axis (X) in the longitudinal direction and which defines a passage (10) therein, said passage (10) having rearward and forward openings (132,131) which are disposed opposite to each other in the longitudinal direction, said inner surrounding barrel surface (13) including a larger-diameter portion (11) and a smaller-diameter portion (12) which are disposed proximate to said rearward and forward openings (132,131), respectively, said larger-diameter portion (11) having a retaining area (16) which is spaced apart from said smaller-diameter portion (12) in the longitudinal direction;
a tubular grip member (3) which, in the position of use, is disposed to bring said gripped portion (212) into engagement with said retaining area (16) by virtue of a first frictional force generated therebetween; and
a tubular plunger (4) which is disposed to be movable in said passage (10) along said larger-diameter portion (11), said plunger (4) having a front opened end wall (421) which is movable to abut against said grip member (3), a rear opened end wall (422) which is disposed opposite to said front opened end wall (421) and which extends outwardly of said rearward opening (132) so as to be manually operable, and an intermediate surrounding wall (42) which is interposed between said front and rear opened end walls (421,422) and which defines an accommodation chamber (41),
said smaller-diameter portion (12) including a retaining region (121) which is disposed proximate to said larger-diameter portion (11) and which is configured to retain said front hub portion (211) thereat when said needle seat (21) is in a position of use, the disposable syringe further comprising
a coupling member (51) having a surrounding retained portion (512) which surrounds the axis (X) and which is disposed in said accommodation chamber (41) to be in frictional engagement with said intermediate surrounding wall (42) by virtue of a second frictional force, and an anchored portion (514) which is disposed adjacent to said front opened end wall (421) in the position of use, which confronts said rear anchoring portion (213); and
a biasing member (52, 54) disposed to bias said anchored portion (514) towards a retracted position where said anchored portion (514) is disposed closer to said rear opened end wall (422) and where said needle seat (21) and said needle cannula (22) are received in said accommodation chamber (41), **characterized in that** said smaller diameter portion (12) includes a friction diminishing region (122) which extends from said retaining region (121) toward said forward opening (131) and which terminates at a shoulder abutment (124), said shoulder abutment (124) defining a communicating hole (123) which permits passage of said needle cannula (22) therethrough, and being spaced apart from said front hub portion (211) along the axis (X) in the position of use and said coupling member (51) is engageable with said rear anchoring portion (213) by a holding force when said coupling member (51) is moved forwardly towards said forward opening (131) such that, when said grip member (3) is pushed forward by virtue of forward movement of said plunger (4) against the first frictional force to cause said front hub portion (211) to move past said friction diminishing region to abut against said shoulder abutment (124) a pushing force subsequently applied to said plunger (4) causes said anchored portion (514) to rub against said rear anchoring portion (213), which remains unmoved in place due to engagement of said front hub portion (211) with said shoulder abutment (124), so that said anchored portion (514) is engaged with said rear anchoring portion (213) and is moved relative to said intermediate surrounding wall (42) towards said rear opened end wall (422) so as to release said surrounding retained portion (512) from said intermediate surrounding wall (42), thereby enabling said anchored portion (514) to be moved from the position of use to said retracted position.

2. The disposable syringe of Claim 1, **characterized in that** said passage (10) at said friction diminishing region (122) has a diameter larger than that of said passage (10) at said retaining region (121).

3. The disposable syringe of Claim 1, **characterized in that** said tubular needle seat (21) is in form of a metal tube which includes a front segment (211) to serve as said front hub portion (211), and a rear segment that is opposite to said front segment (211) in the longitudinal direction and that has outer and inner segment surfaces opposite to each other in radial directions relative to the axis (X), said outer and inner segment surfaces being configured to serve as said gripped portion (212) and said rear anchoring portion (213), respectively.

4. The disposable syringe of Claim 1, **characterized in that** said intermediate surrounding wall (42) of said tubular plunger (4) has a smaller front segment (423) and a larger rear segment (424) disposed proximate to said front and rear opened end walls (421,422), respectively, and an enlarged terminal segment (425) disposed between said larger rear segment (424) and said rear opened end wall (422) .

5. The disposable syringe of Claim 4, **characterized in that** said enlarged terminal segment (425) has a vent hole (429) in fluid communication with the ambient atmosphere.

6. The disposable syringe of Claim 5, **characterized in that** said coupling member (51) has a shank portion (511) which extends from said surrounding retained portion (512) distal from said anchored portion (514), said biasing member (52) including a coiled spring (52) which has a front spring end (521) that engages said shank portion (511) , and a rear spring end (522) that is retained to said rear opened end wall (422) such that said coiled spring (52) is tensioned when said surrounding retained portion (512) is in frictional engagement with said intermediate surrounding wall (42) by virtue of the second frictional force.

7. The disposable syringe of Claim 6, **characterized in that** said larger rear segment (424) has an annular rib (428) which is disposed thereon proximate to said rear opened end wall (422), said rear spring end (522) of said coiled spring (52) being retained by said annular rib (428).

8. The disposable syringe of Claim 5, **characterized in that** said coupl ing member (51) has a shank portion (511) which is interposed between said anchored portion (514) and said surrounding retained portion (512), said intermediate surrounding wall (42) and said shank portion (511) respectively having an annular shoulder (426) and a flange (515) which are respectively proximate to said front opened end wall (421) and distal from said anchored portion (514) and which are spaced apart from each other in the longitudinal direction so as to define a biasing member receiving space therebetween, said biasing member (54) being a coiled spring (54) which has front and rear spring ends (541,542) abutting against said annular shoulder (426) and said flange (515), respectively, such that said coiled spring (54) is compressed when said surrounding retained portion (512) is in frictional engagement with said intermediate surrounding wall (42) by virtue of the second frictional force.

9. The disposable syringe of Claim 1, **characterized in that** said rear anchoring portion (213) extends rearwardly from said gripped portion (212) along the axis (X), said anchored portion (514) having an engaging recess (516) which is configured to grip said rear anchoring portion (213) with the holding force when said needle seat (21) is to be placed in the retracted position.

10. The disposable syringe of Claim 1, further **characterized by** an end cap (44) disposed to cover said rear opened end wall (422), and a seal ring member (45) which is disposed to establish fluid-tight engagement between said end cap (44) and said rear opened end wall (422).

11. The disposable syringe of Claim 10, **characterized in that** said biasing member includes a fluid which is contained in said accommodation chamber (41) at a reduced pressure, and a sealing member (55) which is configured to provide a sealing between said coupling member (51) and said intermediate surrounding wall (42) so as to trap said fluid in said accommodation chamber (41) such that when said surrounding retained portion (512) is released from said intermediate surrounding wall (42), said anchoredportion (514) is suctioned to the retracted position due to a pressure difference between the ambient atmosphere and the reduced pressure.

12. The disposable syringe of Claim 11, **characterized in that** said sealing member (55) extends in the longitudinal direction to terminate at a rear end wall (550) which is disposed rearwardly of said coupling member (51) and which has a central recess (551) extending inwardly and along the axis (X) so as to enhance deformability of said rear end wall (550) in radial directions, thereby enhancing sealing attachment of said sealing member (55) with said intermediate surrounding wall (42).

13. The disposable syringe of Claim 11, **characterized in that** said coupling member (51) has a shank portion (511) extending rearwardly from said surrounding retained portion (512) to terminate at an enlarged head portion (519), said sealing member (55) being sleeved on said shank portion (511) and being interposed between said head portion (519) and said surrounding retained portion (512) so as to be deformed radially for enhancing sealing attachment of said sealing member (55) with said intermediate surrounding wall (42).

14. The disposable syringe of Claim 11, **characterized in that** said sealing member (55) surrounds the axis (X), and is formed integrally with said coupling member (51) .

15. The disposable syringe of Claim 11, **characterized in that** said sealing member (55) is formed integrally with said coupling member (51), is made from a deformable material, and surrounds said anchored portion (514), said coupling member (51) having an annular front edge (518) which is slidable on and is in fluid-tight engagement with said larger-diameter portion (11) of said inner surrounding barrel surface (13).

16. The disposable syringe of Claim 1, further **characterized by** a sealing unit (7) which is in fluid-tight engagement with said frictiondiminishing region (122) , saidtubular grip member (3) being formed with a deformable sealing portion (32) which is in fluid-tight engagement with said gripped portion (212) of said needle seat (21) so as to form a fluid-tight compressible chamber (73), said compressible chamber (73) being filled with a fluid, said front hub portion (211) of said needle seat (21) having a plurality of through holes (214) formed therethrough so as to be in fluid communication with said compressible chamber (73), such that when said tubular grip member (3) is pushed forwardly by virtue of forward movement of said plunger (4) to move said front hub portion (211) past said friction diminishing region (122), said fluid in said compressible chamber (73) is squeezed through said through holes (214), thereby generating a pressure force in the longitudinal direction that depresses said surrounding gripped portion (212) rearwardly to help thrust said anchored portion (514) to the retracted position.

17. The disposable syringe of Claim 1, **characterized in that** said inner surrounding barrel surface (13) of said barrel (1) further includes an annular shoulder (14) disposed between said larger-diameter portion (11) and said smaller-diameter portion (12), and a plurality of ribs (15) formed on said annular shoulder (14).

18. The disposable syringe of Claim 1, **characterized in that** said coupling member (51) further has a retained shank portion (513) which is disposed between said surrounding retained portion (512) and said anchored portion (514) and which extends in said accommodation chamber (41) at said smaller front segment (423) so as to stabilize said coupling member (51) at said smaller front segment (423) .

## Patentansprüche

1. Einwegspritze, die Folgendes umfasst:
eine Nadelkanüle (22),
einen röhrenförmigen Nadelsitz (21), der einen vorderen Verbindungsstückabschnitt (211), der dafür angeordnet ist, die Nadelkanüle (22) in demselben zu befestigen, einen ergriffenen Abschnitt (212), der sich von dem vorderen Verbindungsstückabschnitt (211) aus in einer Längsrichtung erstreckt, und einen hinteren Verankerungsabschnitt (213), der entgegengesetzt zu dem vorderen Verbindungsstückabschnitt (211) in der Längsrichtung angeordnet ist, einschließt,
einen Zylinder (1), der eine innere umschließende Zylinderfläche (13) hat, die eine Achse (X) in der Längsrichtung umschließt und die darin einen Durchgang (10) definiert, wobei der Durchgang (10) eine hintere und eine vordere Öffnung (132, 131) hat, die entgegengesetzt zueinander in der Längsrichtung angeordnet sind, wobei die innere umschließende Zylinderfläche (13) einen Abschnitt mit größerem Durchmesser (11) und einen Abschnitt mit kleinerem Durchmesser (12), die nahe der hinteren bzw. der vorderen Öffnung (132, 131) angeordnet sind, einschließt, wobei der Abschnitt mit größerem Durchmesser (11) eine Rückhaltefläche (16) hat, die mit Zwischenraum zu dem Abschnitt mit kleinerem Durchmesser (12) in der Längsrichtung angeordnet ist,
ein röhrenförmiges Greifelement (3), das in der Anwendungsstellung dafür angeordnet ist, den ergriffenen Abschnitt (212) auf Grund einer zwischen denselben erzeugten ersten Reibungskraft in Eingriff mit der Rückhaltefläche (16) zu bringen, und
einen röhrenförmigen Druckkolben (4), der dafür angeordnet ist, in dem Durchgang (10) längs des Abschnitts mit größerem Durchmesser (11) bewegt werden zu können, wobei der Druckkolben (4) eine vordere geöffnete Stirnwand (421), die bewegt werden kann, um an das Greifelement (3) anzustoßen, eine hintere geöffnete Stirnwand (422), die entgegengesetzt zu der vorderen geöffneten Stirnwand (421) angeordnet ist und die sich von der hinteren Öffnung (132) aus nach außen erstreckt, um so von Hand betrieben werden zu können, und eine umschließende Zwischenwand (42), die zwischen der vorderen und der hinteren geöffneten Stirnwand (421, 422) angeordnet ist und die eine Aufnahmekammer (41) definiert, hat,
wobei der Abschnitt mit kleinerem Durchmesser (12) einen Rückhaltebereich (121) einschließt, der nahe dem Abschnitt mit größerem Durchmesser (11) angeordnet ist und der dafür konfiguriert ist, den vorderen Verbindungsstückabschnitt (211) daran zurückzuhalten, wenn sich der Nadelsitz (21) in einer Anwendungsstellung befindet, wobei die Einwegspritze ferner Folgendes umfasst:
ein Kupplungselement (51), das einen umschließenden zurückgehaltenen Abschnitt (512), der die Achse (X) umschließt und der in der Aufnahmekammer (41) dafür angeordnet ist, sich auf Grund einer zweiten Reibungskraft in Reibungseingriff mit der umschließenden Zwischenwand (42) zu befinden, und einen verankerten Abschnitt (514), der in der Anwendungsstellung angrenzend an die vordere geöffnete Stirnwand (421) angeordnet ist, der dem hinteren Verankerungsabschnitt (213) gegenüberliegt, und,
ein Vorspannelement (52, 54), das dafür angeordnet ist, den verankerten Abschnitt (514) zu einer eingezogenen Stellung hin vorzuspannen, wobei der verankerte Abschnitt (514) näher an der hinteren geöffneten Stirnwand (422) angeordnet ist und wobei der Nadelsitz (21) und die Nadelkanüle (22) in der Aufnahmekammer (41) aufgenommen werden,
**dadurch gekennzeichnet, dass** der Abschnitt mit kleinerem Durchmesser (12) einen Reibungsminderungsbereich (122) einschließt, der sich von dem Rückhaltebereich (121) zu der vorderen Öffnung (131) hin erstreckt und der an einem Absatzwiderlager (124) endet, wobei das Absatzwiderlager (124) ein Verbindungsloch (123) definiert, dass einen Durchgang der Nadelkanüle (22) durch dasselbe ermöglicht, und in der Anwendungsstellung mit Zwischenraum längs der Achse (X) entfernt von den vorderen Verbindungsstückabschnitt (211) angeordnet ist, und das Kupplungselement (51) durch eine Haltekraft mit dem hinteren Verankerungsabschnitt (213) in Eingriff gebracht werden kann, wenn das Kupplungselement (51) vorwärts zu der vorderen Öffnung (131) hin bewegt wird derart, dass, wenn das Greifelement (3) auf Grund einer Vorwärtsbewegung des Druckkolbens (4) gegen die erste Reibungskraft nach vorn geschoben wird, um zu bewirken, dass sich der vordere Verbindungsstückabschnitt (211) an dem Reibungsminderungsbereich vorbei bewegt, um an das Absatzwiderlager (124) anzustoßen, eine anschließend auf den Druckkolben (4) ausgeübte Schubkraft bewirkt, dass der verankerte Abschnitt (514) an dem hinteren Verankerungsabschnitt (213) reibt, der auf Grund des Eingriffs des vorderen Verbindungsstückabschnitts (211) mit dem Absatzwiderlager (124) unbewegt an seinem Platz bleibt, so dass der verankerte Abschnitt (514) mit dem hinteren Verankerungsabschnitt (213) in Eingriff gebracht wird und im Verhältnis zu der umschließenden Zwischenwand (42) zu der hinteren geöffneten Stirnwand (422) hin bewegt wird, um so den umschließenden zurückgehaltenen Abschnitt (512) von der umschließenden Zwischenwand (42) zu lösen, wodurch ermöglicht wird, dass der verankerte Abschnitt (514) von der Anwendungsstellung zu der eingezogenen Stellung bewegt wird.

2. Einwegspritze nach Anspruch 1, **dadurch gekennzeichnet, dass** der Durchgang (10) an dem Reibungsminderungsbereich (122) einen Durchmesser hat, der größer ist als derjenige des Durchgangs (10) an dem Rückhaltebereich (121).

3. Einwegspritze nach Anspruch 1, **dadurch gekennzeichnet, dass** der Nadelsitz (21) die Form einer Metallröhre hat, die ein vorderes Segment (211), das als der vordere Verbindungsstückabschnitt (211) dient, und ein hinteres Segment, das dem vorderen Segment (211) in der Längsrichtung gegenüberliegt und das eine äußere und eine innere Segmentfläche hat, die einander im Verhältnis zu der Achse (X) in Radialrichtungen gegenüberliegen, einschließt, wobei die äußere und die innere Segmentfläche dafür konfiguriert sind, als der ergriffene Abschnitt (212) bzw. der hintere Verankerungsabschnitt (213) zu dienen.

4. Einwegspritze nach Anspruch 1, **dadurch gekennzeichnet, dass** die umschließende Zwischenwand (42) des röhrenförmigen Druckkolbens (4) ein kleineres vorderes Segment (423) und ein größeres hinteres Segment (424), die nahe der vorderen bzw. der hinteren geöffneten Stirnwand (421, 422) angeordnet sind, und ein vergrößertes Endsegment (425), das zwischen dem größeren hinteren Segment (424) und der hinteren geöffneten Stirnwand (422) angeordnet ist, hat.

5. Einwegspritze nach Anspruch 4, **dadurch gekennzeichnet, dass** das vergrößerte Endsegment (425) ein Lüftungsloch (429) in Fluidverbindung mit der umgebenden Atmosphäre hat.

6. Einwegspritze nach Anspruch 5, **dadurch gekennzeichnet, dass** das Kupplungselement (51) einen Schaftabschnitt (511) hat, der sich distal von dem verankerten Abschnitt (514) von dem umschließenden zurückgehaltenen Abschnitt (512) aus erstreckt, wobei das Vorspannelement (52) eine Schraubenfeder (52) einschließt, die ein vorderes Federende (521), das den Schaftabschnitt (511) in Eingriff nimmt, und ein hinteres Federende (522), das an der hinteren geöffneten Stirnwand (422) zurückgehalten wird, hat, derart, dass die Schraubenfeder (52) gespannt ist, wenn sich der umschließende zurückgehaltene Abschnitt (512) auf Grund der zweiten Reibungskraft in Reibungseingriff mit der umschließenden Zwischenwand (42) befindet.

7. Einwegspritze nach Anspruch 6, **dadurch gekennzeichnet, dass** das größere hintere Segment (424) eine ringförmige Rippe (428) hat, die nahe der hinteren geöffneten Stirnwand (422) an demselben angeordnet ist, wobei das hintere Federende (522) der Schraubenfeder (52) durch die ringförmige Rippe (428) zurückgehalten wird.

8. Einwegspritze nach Anspruch 5, **dadurch gekennzeichnet, dass** das Kupplungselement (51) einen Schaftabschnitt (511) hat, der zwischen dem verankerten Abschnitt (514) und dem umschließenden zurückgehaltenen Abschnitt (512) angeordnet ist, wobei die umschließende Zwischenwand (42) und der Schaftabschnitt (511) jeweils einen ringförmigen Absatz (426) und einen Flansch (515) haben, die sich jeweils nahe der vorderen geöffneten Stirnwand (421) und distal von dem verankerten Abschnitt (514) befinden und die in der Längsrichtung mit Zwischenraum entfernt voneinander angeordnet sind, um so einen Vorspannelement-Aufnahmeraum zwischen denselben zu definieren, wobei das Vorspannelement (54) eine Schraubenfeder (54) einschließt, die ein vorderes und ein hinteres Federende (541, 542), die an den ringförmigen Absatz (426) bzw. den Flansch (515) anstoßen, hat, derart, dass die Schraubenfeder (54) zusammengedrückt ist, wenn sich der umschließende zurückgehaltene Abschnitt (512) auf Grund der zweiten Reibungskraft in Reibungseingriff mit der umschließenden Zwischenwand (42) befindet.

9. Einwegspritze nach Anspruch 1, **dadurch gekennzeichnet, dass** sich der hintere Verankerungsabschnitt (213) längs der Achse (X) von dem ergriffenen Abschnitt (212) aus nach hinten erstreckt, wobei der verankerte Abschnitt (514) eine Eingriffsaussparung (516) hat, die dafür konfiguriert ist, den hinteren Verankerungsabschnitt (413) mit der Haltekraft zu ergreifen, wenn der Nadelsitz (21) in die eingezogene Stellung gebracht werden soll.

10. Einwegspritze nach Anspruch 1, ferner **gekennzeichnet durch** eine Endkappe (44), die dafür angeordnet ist, die hintere geöffnete Stirnwand (422) abzudecken, und ein Dichtungsringelement (45), das dafür angeordnet ist, einen fluiddichten Eingriff zwischen der Endkappe (44) und der hinteren geöffneten Stirnwand (422) herzustellen.

11. Einwegspritze nach Anspruch 10, **dadurch gekennzeichnet, dass** das Vorspannelement ein Fluid, das mit einem verringerten Druck in der Aufnahmekammer (41) enthalten ist, und ein Abdichtungselement (55), das dafür konfiguriert ist, eine Abdichtung zwischen dem Kupplungselement (51) und der umschließenden Zwischenwand (42) bereitzustellen, einschließt, um so das Fluid in der Aufnahmekammer (41) derart einzuschließen, dass, wenn der umschließende zurückgehaltene Abschnitt (512) von der umschließenden Zwischenwand (42) gelöst wird, der verankerte Abschnitt (514) auf Grund eines Druckunterschiedes zwischen der umgebenden Atmosphäre und dem verringerten Druck zu der eingezogenen Stellung gesaugt wird.

12. Einwegspritze nach Anspruch 11, **dadurch gekennzeichnet, dass** sich das Abdichtungselement (55) in der Längsrichtung erstreckt, um an einer hinteren Stirnwand (550) zu enden, die von dem Kupplungselement (51) aus nach hinten angeordnet ist und die eine Mittelaussparung (551) hat, die sich nach innen und längs der Achse (X) erstreckt, um so die Verformbarkeit der hinteren Stirnwand (550) in Radialrichtungen zu verbessern, wodurch die abdichtende Befestigung des Abdichtungselements (55) mit der umschließenden Zwischenwand (42) verbessert wird.

13. Einwegspritze nach Anspruch 11, **dadurch gekennzeichnet, dass** das Kupplungselement (51) einen Schaftabschnitt (511) hat, der sich von dem umschließenden zurückgehaltenen Abschnitt (512) aus nach hinten erstreckt, um an einem vergrößerten Kopfabschnitt (519) zu enden, wobei das Abdichtungselement (55) auf den Schaftabschnitt (511) aufgeschoben ist und zwischen dem Kopfabschnitt (519) und dem umschließenden zurückgehaltenen Abschnitt (512) angeordnet ist, um so in Radialrichtung verformt zu werden, um die abdichtende Befestigung des Abdichtungselements (55) mit der umschließenden Zwischenwand (42) zu verbessern.

14. Einwegspritze nach Anspruch 11, **dadurch gekennzeichnet, dass** das Abdichtungselement (55) die Achse (X) umschließt und integral mit dem Kupplungselement (51) geformt ist.

15. Einwegspritze nach Anspruch 11, **dadurch gekennzeichnet, dass** das Abdichtungselement (55) integral mit dem Kupplungselement (51) geformt ist, aus einem verformbaren Material hergestellt ist und den verankerten Abschnitt (514) umschließt, wobei das Kupplungselement (51) eine ringförmige Vorderkante (518) hat, die auf dem Abschnitt mit größerem Durchmesser (11) der inneren umschließenden Zylinderfläche (13) verschoben werden kann und sich in fluiddichtem Eingriff mit demselben befindet.

16. Einwegspritze nach Anspruch 1, ferner **gekennzeichnet durch** eine Abdichtungseinheit (7), die sich in fluiddichtem Eingriff mit dem Reibungsminderungsbereich (122) befindet, wobei das röhrenförmige Greifelement (3) mit einem verformbaren Abdichtungsabschnitt (32) geformt ist, der sich in fluiddichtem Eingriff mit dem ergriffenen Abschnitt (212) des Nadelsitzes (21) befindet, um so eine fluiddichte zusammendrückbare Kammer (73) zu bilden, wobei die zusammendrückbare Kammer (73) mit einem Fluid gefüllt ist, wobei der vordere Verbindungsstückabschnitt (211) des Nadelsitzes (21) mehrere **durch** denselben geformte Durchgangslöcher (214) hat, um so in Fluidverbindung mit der zusammendrückbaren Kammer (73) zu stehen derart, dass, wenn das röhrenförmige Greifelement (3) auf Grund einer Vorwärtsbewegung des Druckkolbens (4) nach vorn geschoben wird, um den vorderen Verbindungsstückabschnitt (211) an dem Reibungsminderungsbereich (122) vorbei zu bewegen, das Fluid in der zusammendrückbaren Kammer (73) **durch** die Durchgangslöcher (214) gequetscht wird, wodurch eine Druckkraft in der Längsrichtung erzeugt wird, die den umschließenden ergriffenen Abschnitt (212) rückwärts niederdrückt, um dazu beizutragen, den verankerten Abschnitt (514) zu der eingezogenen Stellung zu drängen.

17. Einwegspritze nach Anspruch 1, **dadurch gekennzeichnet, dass** die innere umschließende Zylinderfläche (13) des Zylinders (1) ferner einen ringförmigen Absatz (14), der zwischen dem Abschnitt mit größerem Durchmesser (11) und dem Abschnitt mit kleinerem Durchmesser (12) angeordnet ist, und mehrere Rippen (15), die an dem ringförmigen Absatz (14) geformt sind, einschließt.

18. Einwegspritze nach Anspruch 1, **dadurch gekennzeichnet, dass** das Kupplungselement (51) ferner einen zurückgehaltenen Schaftabschnitt (513) hat, der zwischen dem umschließenden zurückgehaltenen Abschnitt (512) und dem verankerten Abschnitt (514) angeordnet ist und der sich in der Aufnahmekammer (41) an dem kleineren vorderen Segment (423) erstreckt, um so das Kupplungselement (51) an dem kleineren vorderen Segment (423) zu stabilisieren.

## Revendications

1. Seringue jetable, comprenant :
une canule d'aiguille (22) ;
un siège d'aiguille tubulaire (21) englobant une partie de moyeu avant (211) destinée à y fixer ladite canule d'aiguille (22), une partie à préhension (212), s'étendant à partir de ladite partie de moyeu avant (211) dans une direction longitudinale, et une partie d'ancrage arrière (213) agencée en un point opposé à ladite partie de moyeu avant (211) dans la direction longitudinale ;
un cylindre (1) comportant une surface de cylindre interne environnante (13) entourant un axe (X) dans la direction longitudinale, et y définissant un passage (10), ledit passage (10) comportant des ouvertures arrière et avant (132, 131) agencées en des points mutuellement opposés dans la direction longitudinale, ladite surface interne environnante du cylindre (13) englobant une partie à diamètre accru (11) et une partie à diamètre réduit (12), agencées respectivement près desdites ouvertures arrière et avant (132, 131), ladite partie à diamètre accru (11) comportant une zone de retenue (16) espacée de ladite partie à diamètre réduit (12) dans la direction longitudinale ;
un élément de préhension tubulaire (3), agencé dans la position d'utilisation, de sorte à entraîner l'engagement de ladite partie à préhension (212) dans ladite zone de retenue (16), par l'intermédiaire d'une première force de frottement produite entre elles ; et
un piston tubulaire (4) agencé de sorte à pouvoir être déplacé dans ledit passage (10) le long de ladite partie à diamètre accru (11), ledit piston (4) comportant une paroi d'extrémité avant ouverte (421), pouvant être déplacée de sorte à buter contre ledit élément de préhension (3), une paroi d'extrémité arrière ouverte (422) agencée en un point opposé à ladite paroi d'extrémité avant ouverte (421) et s'étendant vers l'extérieur de ladite ouverture arrière (132), de sorte à pouvoir être actionnée manuellement, et une paroi intermédiaire environnante (42) agencée entre lesdites parois d'extrémité avant et arrière ouvertes (421, 422), et définissant une chambre de réception (41) ;
ladite partie à diamètre réduit (12) englobant une région de retenue (121) agencée près de ladite partie à diamètre accru (11) et configurée de sorte à y retenir ladite partie de moyeu avant (211) lorsque ledit siège de l'aiguille (21) se trouve dans une position d'utilisation, la seringue jetable comprenant en outre :
un élément d'accouplement (51) comportant une partie environnante à retenue (512), entourant l'axe (X) et agencée dans ladite chambre de réception (41) de sorte à être engagée par frottement dans ladite paroi intermédiaire environnante (42), par l'intermédiaire d'une deuxième force de frottement, et une partie à ancrage (514) agencée près de ladite paroi d'extrémité avant ouverte (421) dans la position d'utilisation, opposée à ladite partie d'ancrage arrière (213) ; et
un élément poussoir (52, 54) destiné à pousser ladite partie à ancrage (514) vers une position rétractée dans laquelle ladite partie à ancrage (514) est agencée plus près de ladite paroi d'extrémité arrière ouverte (422), ledit siège d'aiguille (21) et ladite canule d'aiguille (22) étant reçus dans ladite chambre de réception (41).
**caractérisée en ce que** ladite partie à diamètre réduit (12) englobe une région à réduction du frottement (122) s'étendant à partir de ladite région de retenue (121) vers ladite ouverture avant (131) et se terminant au niveau d'une butée d'épaulement (124), ladite butée d'épaulement (124) définissant un trou de communication (123) permettant le passage de ladite canule d'aiguille (22), et étant espacée de ladite partie de moyeu avant (211) le long de l'axe (X) dans la position d'utilisation, ledit élément d'accouplement (51) pouvant s'engager dans ladite partie d'ancrage arrière (213) par l'intermédiaire d'une force de retenue, lors du déplacement vers l'avant dudit élément d'accouplement (51) vers ladite ouverture avant (131), de sorte que lorsque ledit élément de préhension (3) est poussé vers l'avant par suite d'un déplacement vers l'avant dudit piston (4), contre la première force de frottement, pour entraîner le déplacement de ladite partie de moyeu avant (211) au-delà de ladite région à réduction du frottement, en vue de sa butée contre ladite butée d'épaulement (124), une force de poussée appliquée ultérieurement audit piston (4) entraîne le frottement de ladite partie à ancrage (514) contre ladite partie d'ancrage arrière (213), qui ne bouge pas de sa position par suite de l'engagement de ladite partie de moyeu avant (211) dans ladite butée d'épaulement (124), ladite partie à ancrage (514) s'engageant ainsi dans ladite partie d'ancrage arrière (213) et étant déplacée par rapport à ladite paroi environnante intermédiaire (42), vers ladite paroi d'extrémité ouverte arrière (422), de sorte à dégager ladite partie environnante à retenue (512) de ladite paroi environnante intermédiaire (42), permettant ainsi le déplacement de ladite partie ancrée (514) à partir de ladite position d'utilisation vers ladite position rétractée ; et

2. Seringue jetable selon la revendication 1, **caractérisée en ce que** ledit passage (10) au niveau de ladite région à réduction du frottement (122) a un diamètre supérieur à celui dudit passage (10) au niveau de ladite région de retenue (121).

3. Seringue jetable selon la revendication 1, **caractérisée en ce que** ledit siège d'aiguille tubulaire (21) a la forme d'un tube métallique englobant un segment avant (211) destiné à établir ladite partie de moyeu avant (211), et un segment arrière opposé audit segment avant (211) dans la direction longitudinale, et comportant des surfaces de segment externe et interne mutuellement opposées dans des directions radiales par rapport à l'axe (X), lesdites surfaces de segment externe et interne du segment étant configurées de sorte à établir respectivement ladite partie à préhension (212) et ladite partie d'ancrage arrière (213).

4. Seringue jetable selon la revendication 1, **caractérisée en ce que** ladite paroi intermédiaire environnante (42) dudit piston tubulaire (4) comporte un segment avant plus petit (423) et un segment arrière plus grand (424) agencés respectivement près desdites parois d'extrémité avant et arrière ouvertes (421, 422), et un segment terminal agrandi (425) agencé entre ledit segment arrière plus grand (424) et ladite paroi d'extrémité arrière ouverte (422).

5. Seringue jetable selon la revendication 4, **caractérisée en ce que** ledit segment terminal agrandi (425) comporte un trou d'évent (429), en communication de fluide avec l'atmosphère ambiante.

6. Seringue jetable selon la revendication 5, **caractérisée en ce que** ledit élément d'accouplement (51) comporte une partie de tige (511) s'étendant à partir de ladite partie à retenue environnante (512), dans une direction distale par rapport à ladite partie à ancrage (514), ledit élément poussoir (52) englobant un ressort hélicoïdal (52) comportant une extrémité de ressort avant (521) s'engageant dans ladite partie de tige (511), et une extrémité de ressort arrière (522) retenue sur ladite paroi d'extrémité arrière ouverte (422), de sorte que ledit ressort hélicoïdal (52) est tendu lorsque ladite partie à retenue environnante (512) est engagée par frottement dans ladite paroi intermédiaire environnante (42), par l'intermédiaire de la deuxième force de frottement.

7. Seringue jetable selon la revendication 6, **caractérisée en ce que** ledit segment arrière plus grand (424) comporte une nervure annulaire (428) qui y est agencée, près de ladite paroi d'extrémité arrière ouverte (422), ladite extrémité arrière de ressort (522) dudit ressort hélicoïdal (52) étant retenue par ladite nervure annulaire (428).

8. Seringue jetable selon la revendication 5, **caractérisée en ce que** ledit élément d'accouplement (51) comporte une partie de tige (511) agencée entre ladite partie à ancrage (514) et ladite partie à retenue environnante (512), ladite paroi intermédiaire environnante (42) et ladite partie de tige (511) comportant respectivement un épaulement annulaire (426) et une bride (515), agencés respectivement près de ladite paroi d'extrémité avant ouverte (421) et dans une direction distale par rapport à ladite partie à ancrage (514), et espacés l'un de l'autre dans la direction longitudinale, de sorte à définir un espace de réception de l'élément poussoir entre eux, ledit élément poussoir (54) étant constitué par un ressort hélicoïdal (54) comportant des extrémités de ressort avant et arrière (541, 542) butant respectivement contre ledit épaulement annulaire (426) et ladite bride (515), de sorte que ledit ressort hélicoïdal (54) est comprimé lorsque ladite partie à retenue environnante (512) est engagée par frottement dans ladite paroi environnante intermédiaire (42), par l'intermédiaire de la deuxième force de frottement.

9. Seringue jetable selon la revendication 1, **caractérisée en ce que** ladite partie d'ancrage arrière (213) s'étend vers l'arrière à partir de ladite partie à préhension (212), le long de l'axe (X), ladite partie à ancrage (514) comportant un évidement d'engagement (516) configuré de sorte à saisir ladite partie d'ancrage arrière (213) par l'intermédiaire de la force de retenue lorsque ledit siège de l'aiguille (21) doit être placé dans la position rétractée.

10. Seringue jetable selon la revendication 1, **caractérisée en outre par** un capuchon d'extrémité (44) destiné à recouvrir ladite paroi d'extrémité arrière ouverte (422), et un élément de bague d'étanchéité (45) destiné à établir un engagement étanche au fluide entre ledit capuchon d'extrémité (44) et ladite paroi d'extrémité arrière ouverte (422).

11. Seringue jetable selon la revendication 10, **caractérisée en ce que** ledit élément poussoir englobe un fluide contenu dans ladite chambre de réception (41), en présence d'une pression réduite, et un élément d'étanchéité (55) destiné à établir l'étanchéité entre ledit élément d'accouplement (51) et ladite paroi intermédiaire environnante (42), pour piéger ledit fluide dans ladite chambre de réception (41), de sorte que lorsque ladite partie à retenue environnante (512) est dégagée de ladite paroi intermédiaire environnante, ladite partie à retenue (514) est entraînée par aspiration vers la position rétractée, par suite d'une différence de pression entre l'atmosphère ambiante et la pression réduite.

12. Seringue jetable selon la revendication 11, **caractérisée en ce que** ledit élément d'étanchéité (55) s'étend dans la direction longitudinale et se termine au niveau d'une paroi d'extrémité arrière (550) agencée vers l'arrière dudit élément d'accouplement (51) et comportant un évidement central (551) s'étendant vers l'intérieur et le long de l'axe (X), pour accroître le pouvoir de déformation de ladite paroi d'extrémité arrière (550) dans des directions radiales, renforçant ainsi la fixation étanche dudit élément d'étanchéité (55) sur ladite paroi intermédiaire environnante (42).

13. Seringue jetable selon la revendication 11, **caractérisée en ce que** ledit élément d'accouplement (51) comporte une partie de tige (511) s'étendant vers l'arrière à partir de ladite partie à retenue environnante (512) et se terminant au niveau d'une partie de tête agrandie (519), ledit élément d'étanchéité (55) étant emmanché sur ladite partie de tige (511) et étant agencé entre ladite partie de tête (519) et ladite partie à retenue environnante (512), de sorte à être déformé radialement, pour renforcer la fixation étanche dudit élément d'étanchéité (55) sur ladite paroi intermédiaire environnante (42).

14. Seringue jetable selon la revendication 11, **caractérisée en ce que** ledit élément d'étanchéité (55) entoure l'axe (X) et est formé d'une seule pièce avec ledit élément d'accouplement (51).

15. Seringue jetable selon la revendication 11, **caractérisée en ce que** ledit élément d'étanchéité (55) est formé d'une seule pièce avec ledit élément d'accouplement (51), **en ce qu'**il est composé d'un matériau déformable et entoure ladite partie à ancrage (514), ledit élément d'accouplement (51) comportant un bord annulaire avant (518) pouvant glisser sur ladite partie à diamètre accru (11) de ladite surface interne environnante du cylindre (13) et étant engagé de manière étanche au fluide dans celle-ci.

16. Seringue jetable selon la revendication 1, **caractérisée en outre par** une unité d'étanchéité (7), engagée de manière étanche au fluide dans ladite région à réduction du frottement (122), ledit élément de préhension tubulaire (13) comportant une partie d'étanchéité déformable (32) engagée de manière étanche au fluide dans ladite partie à préhension (212) dudit siège de l'aiguille (21), de sorte à former une chambre pouvant être comprimée de manière étanche au fluide (73), ladite chambre à compression (73) étant remplie d'un fluide, ladite partie de moyeu avant (211) dudit siège de l'aiguille (21) comportant plusieurs trous de passage (214) la traversant, pour établir une communication de fluide avec ladite chambre à compression (73), de sorte que lorsque ledit élément de préhension tubulaire (3) est poussé vers l'avant par suite d'un déplacement vers l'avant dudit piston (4), pour déplacer ladite partie de moyeu avant (211) le long de ladite région à réduction du frottement (122), ledit fluide dans ladite chambre à compression (73) est pressé à travers lesdits trous de passage (214), produisant ainsi une force de pression dans la direction longitudinale, pressant ladite partie à préhension environnante (212) vers l'arrière pour faciliter la poussée de ladite partie à ancrage (514) vers la position rétractée.

17. Seringue jetable selon la revendication 1, **caractérisée en ce que** ladite surface interne environnante du cylindre (13) dudit cylindre (1) englobe en outre un épaulement annulaire (14) agencé entre ladite partie à diamètre accru (11) et ladite partie à diamètre réduit (12), plusieurs nervures (15) étant formées sur ledit épaulement annulaire (14).

18. Seringue jetable selon la revendication 1, **caractérisée en ce que** ledit élément d'accouplement (15) comporte en outre une partie de tige à retenue (513), agencée entre ladite partie à retenue environnante (512) et ladite partie à ancrage (514), et s'étendant dans ladite chambre de réception (41) au niveau dudit segment avant plus petit (423), de sorte à stabiliser ledit élément d'accouplement (51) au niveau dudit segment avant plus petit (423).
